# EUROPÄISCHE PATENTSCHRIFT

(11) **EP 0 647 438 B1**
(45) Veröffentlichungstag und Bekanntmachung des Hinweises auf die Patenterteilung: **12.01.2000**
(21) Anmeldenummer: 94104360.6
(22) Anmeldetag: 19.03.1994
(51) Int. Cl.: A61F 2/06

(54) **Reduzierstent und Vorrichtung mit Reduzierstent**
Reduction stent and device with this stent
Stent à réduction et dispositif avec ce stent

(30) Priorität: 07.10.1993 DE 4334140
(43) Veröffentlichungstag der Anmeldung: 12.04.1995
(73) Patentinhaber: ANGIOMED GmbH & Co. Medizintechnik KG, 76227 Karlsruhe (DE)
(72) Erfinder: Hauenstein, Karlheinz, Hans, Prof. Dr, D-79288 Gottenheim (DE); Lindenberg, Josef, D-76227 Karlsruhe (DE)
(74) Vertreter: Lempert, Jost, Dipl.-Phys. Dr. rer.nat.

(56) Entgegenhaltungen:
- EP-A- 0 481 365
- EP-A- 0 556 850
- EP-A- 0 587 197
- WO-A-93/08767
- US-A- 4 501 263

## Beschreibung

Die Erfindung betrifft im Allgemeinen einen Stent, wie er im Oberbegriff des Anspruchs 1 definiert wird. Ein Solcher Stent ist z.B. aus der EP-A-0481365 bekannt. Insbesondere betrifft die Anmeldung einen Reduzierstent zum Reduzieren der Durchflußöffnung eines Ganges in einem lebendem Körper, wie insbesondere eines transjugulären intrahepatischen portosystemischen Shunts und eine Vorrichtung zum Anlegen eines transjugulären intrahepatischen portosystemischen Shunts.

Bei einer Leberschädigung, insbesondere einer Leberzirrhose, wird intervenöser Blutfluß von der Pfortader durch die Leber zur Lebervene reduziert. Es ergibt sich eine Blutdruckerhöhung auf der Pfortaderseite, die zu Ösophagusvarizen, also Krampfadern im Bereich der Speiseröhre führen kann. Platzen diese auf, so besteht die Gefahr eines Verblutens des Patienten. Es ist bekannt, zur Reduzierung des Blutdrucks eine portokavale, mesocavale oder splenorenale Anastomose vorzunehmen, also eine Verbindung durch Anastomose des Pfortaderstammes und der Vena cava inf. der Darmvene (Venesenterica superior) oder zwischen Milz- und li Nierenvene vorzunehmen. Hierdurch erfolgt eine Druckentlastung bei der beschriebenen portalen Hypertension.

Wäre lediglich eine solche Verbindung (Shunt) gelegt, kann diese sich wieder verschließen. Eine bestimmte Größe des Shuntlumens ist operativ nicht eindeutig vorherbestimmbar. Heute wird der Shunt daher durch Einführen eines Stents in Form eines zylindrischen, vorzugsweise um seine Achse biegbaren Gitter-Stents zuverlässig offengehalten. Im Notfall wird man zur schnellen Senkung des Pfortaderhochdrucks einen Shunt mit erheblichem Durchmesser legen, um eine rasche und ausreichende Drucksenkung und damit Stillstand der Varizenblutung zu erreichen. Das durch diesen Shunt hindurchfließende Blut fließt allerdings ohne Reinigung durch die Leber ins Gehirn, so daß es dort durch die aus dem Blut nicht ausgefilterten Substanzen wie Ammoniak, möglicherweise auch Amine und Phenolkörper zu einer zerebralen Vergiftung und damit einer hepatischen Enzephalopathie und damit der Reduzierung oder erheblichen Beeinträchtigung der Gehirnfunktion des Patienten kommen kann. Auch ist vorab die Größe eines geeigneten Stent-Durchmessers grundsätzlich nicht abzusehen, da die richtig abgegrenzte Größe zwischen relativ großem Durchmesser zur Vermeidung eines Pfortaderhochdrucks einerseits und andererseits eines reduzierten Durchmessers, um möglichst wenig ungereinigte Blut durch die Leber und einen möglichst großen Anteil des Blutes der Leberreinigung unterziehen zu können, von verschiedenen Faktoren, wie beispielsweise der Viskosität des Blutes, abhängt und daher schwer abzustimmen ist.

Ausgehend von dieser Problematik schlägt die Erfindung die nachträgliche Reduzierung des freien Durchflußbereiches des Shunts durch Einlegen eines Reduzierteils und als solches einen Reduzierstent zum Reduzieren des Durchmessers eines solchen durchflossenen Ganges im Körper, wie eines solchen transjugulären intrahepatischen portosystemischen Shunts (TIPS) vor, der einen hülsenartigen Teil mit durchbrochenen Wandungen aufweist, der erweiterte Enden und einen durch eine Einschnürung in seinem Durchmesser reduzierten Zwischenbereich aufweist und auf der Außenseite des hülsenartigen Teils zwischen den erweiterten Enden mit thrombogenen Fäden versehen ist.

Die Erfindung sieht weiterhin eine Vorrichtung zum Anlegen eines transjugulären intrahepatischen portosystemischen Shunts vor, die zum Offenhalten des Shunts einen im wesentlichen zylindrischen Stent relativ großen Durchmessers und einen Reduzierstent zum Reduzieren des hierdurch geschaffenen Durchmessers in der vorstehend beschriebenen Ausgestaltung aufweist. Weiterhin sieht die Erfindung einen Reduzierstent der beschriebenen Art zum Reduzieren des Durchmessers eines flüssigkeitsdurchflossenen Ganges im menschlichen Körper, wie eben eines solchen transjugulären intrahepatischen portosystemischen Shunts.

Gemäß einer bevorzugten Ausgestaltung ist vorgesehen, daß der Reduzierstent selbstexpandierend ist. In weiterer Ausgestaltung kann vorgesehen sein, daß er aus Nitinol besteht und derart vorbehandelt ist, daß er in einer Niedrigtemperaturstellung eine relativ gestreckte Konfiguration geringen Durchmessers aufweist, so daß er durch einen Katheter eingeführt werden kann und in einer Körpertemperaturstellung (Hochtemperaturstellung) sich in die erweiterte beschriebene Form aufstellt. Die Konfiguration in Betriebsstellung bei erhöhter Temperatur kann unterschiedlich sein. So kann der Stent doppelkonusförmig oder als einschaliger Hyper- boloid ausgebildet sein.

Die besprochenen Wandungen können in verschiedenartiger Form ausgestaltet sein. So ist in bevorzugter Ausgestaltung vorgesehen, daß die Wandungen waben- oder gitterartig sind. Alternativ kann das hülsenartige Teil auch gewebt, gewirkt oder gestrickt sein. Die thrombogenen Fäden sind vorzugsweise im wesentlichen achsparallel ausgerichtet. Je nach Stärke der gewünschten Thrombogenität des Fadenmaterials kommen verschiedene Materialien in Frage, wie monofile Kunststoffe, Dacron (ein eingetragenes Warenzeichen), Baumwolle, Seide, Leinen.

Der maximale Durchmesser des Reduzierstents an seinen erweiterten Enden liegt vorzugsweise in der Betriebsstellung geringfügig über dem Durchmesser des zylindrischen, den Shunt bedingenden Stents, so daß der Reduzierstent sich beidseitig überall am Shunt-Stent verhaken kann und damit zuverlässig festgelegt ist. In bevorzugter Ausgestaltung ist weiterhin vorgesehen, daß die Länge des Reduzierstents wesentlich unter der des Shunt-Stents liegt, so daß ggf., falls beispielsweise der Durchfluß eines zunächst gelegten Reduzierstents noch zu groß ist, axial hinter diesem ein weiterer Reduzierstent mit geringerem minimalen Durchmesser eingesetzt werden kann.

Die thrombogenen Fäden im Reduzierstent initiieren eine Blutgerinnung im Außenbereich des Reduzierstents, wodurch der Durchmesser des Kanals effektiv reduziert wird.

Bei vorgegebenem Fadenmaterial kann die Thrombogenität des Stents durch geeignete Wahl der Fadenlänge eingestellt werden.

Weitere Vorteile und Merkmale der Erfindung ergeben sich aus den Ansprüchen und aus der nachfolgenden Beschreibung, in der ein Ausführungsbeispiel der Erfindung unter Bezugnahme auf die Zeichnungen im einzelnen erläutert ist. Dabei zeigt:
- Figur 1a: einen erfindungsgemäßen Reduzierstent in Körpertemperatur- oder Betriebsstellung als Längsschnitt, wobei ein abgestimmter Shunt-Stent gestrichelt angedeutet ist;
- Figur 1b: den Reduzierstent in seiner Tieftemperatur- bzw. Einführstellung, in der er durch einen Katheter zum Anlageort geführt werden kann;
- Figur 2: eine schematische Darstellung einer in einer Leber plazierten erfindungsgemäßen Vorrichtung aus Shunt-Stent und in diesem eingesetzten Reduzierstent, wobei die Kontur des letzteren lediglich gestrichelt angedeutet ist.

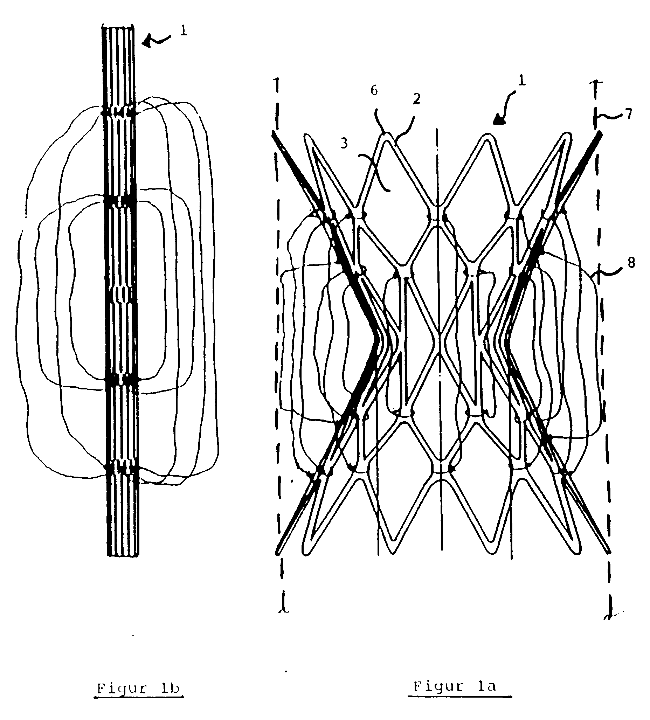

Der erfindungsgemäße Reduzierstent 1 besteht aus einem hülsenartigen Teil 2 mit wabenartigen Durchbrechungen 3 in Form eines Gitters. Er kann hergestellt werden, indem ein Blechteil mit hintereinander und nebeneinander angeordneten kurzen Schlitzen versehen wird, die jeweils einen endlichen Abstand zueinander aufweisen, wobei die Schlitze nebeneinander angeordneter Schlitzreihen in ihrer Richtung versetzt sind, üblicherweise um die halbe Länge eines Schlitzes. Der Reduzierstent 1 ist so vorbehandelt, daß er in seiner Körpertemperatur- oder Einsatzstellung eine doppelkonische Kontur aufweist, d.h. seine beiden Stirnseiten erweitert sind und einen relativ großen Radius aufweisen, während der Mittelbereich mit einer einen geringeren Durchmesser bewirkenden Einschnürung versehen ist. An den Enden sind Spitzen 6 ausgebildet, mittels derer der Reduzierstent 1 sich in einem vorab gelegten Shunt-Stent 7 verhaken kann, wie dies in Figur 1a dargestellt ist, so daß hierdurch eine vollständige Festlegung des Reduszierstents 1 erreicht wird.

Auf seiner Außenseite im Bereich zwischen den erweiterten Enden ist der Reduzierstent mit Fäden 8 aus thrombogenem Material versehen, die beispielsweise, wie dies dargestellt ist, durch Knüpfen an Knotenpunkten des Gitters 2,3 festgelegt sein können. Nach Legen des Reduzierstents bilden sich an den Fäden im Außenbereich des Stents Blutgerinnsel, wodurch der effektive Durchflußbereich auf etwa den minimalen Querschnitt des Stents in seinem Mittelbereich reduziert wird. Hierdurch wird eine effektive Reduzierung des Durchflußlumens erreicht.

In der Figur 1b ist der Reduzierstent 1 in seiner Einführ- oder Tieftemperaturstellung dargestellt. In dieser Stellung kann er durch einen Katheter bis in den Anlagebereich eingeführt werden.

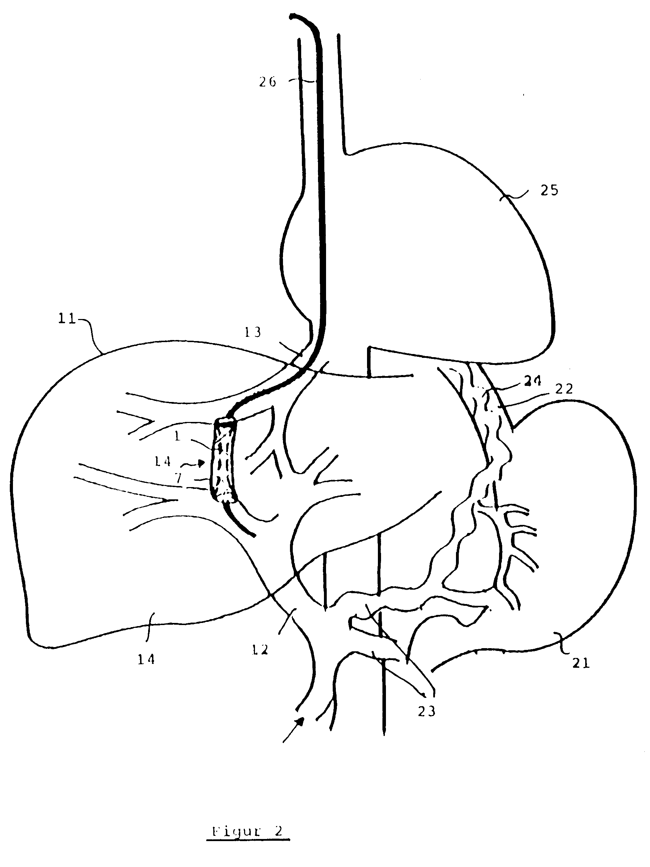

Die Figur 2 zeigt schematisch eine Leber 11 mit Pfortader 12 und Lebervene 13. Beide Venen verästeln sich üblicherweise in das Lebergewebe. Es ist erkennbar, daß von der Pfortader zum Magen 21 und zur Speiseröhre führende Blutgefäße 23 abgehen, die Varizen 24 ausbilden können. Auch sind das Herz 25 und ein vom Hals des Patienten am Herz 25 vorbei bis in eine Lebervene geschobener Einführkatheter 26 erkennbar.

Es ist weiterhin ein Shunt 14 zwischen Pfortader 12 und Lebervene 13 dargestellt, der durch Durchstechen des Lebergewebes hergestellt wurde. In dem Shunt 14 befindet sich zum Offenhalten desselben ein Shunt-Stent 7, in den weiterhin zur Reduzierung des freien Durchflußlumens ein Reduzierstent 1 in der vorstehend beschriebenen Ausgestaltung eingebracht wurde. Der Shuntstent 7 kann grundsätzlich aus dem gleichen Material hergestellt sein wie der Reduzierstent 1 und damit auch Gitter- oder Wabenform aufweisen, wobei das Gitter bzw. die Waben in der gleichen Weise, wie oben beschrieben, erzeugt wurden. Der Shuntstent 7 weist grundsätzlich zylindrische Form auf. Durch wechselweises Auftrennen der Knoten des Gitters oder der Waben wird eine Biegbarkeit der Achse des Shuntstents 7 erreicht, so daß er auch in eine gekrümmte Shunts eingesetzt werden kann.

Der Erfindungsgegenstand kommt folgendermaßen zum Einsatz. Zunächst wird durch Punktion mittels einer Punktionskanüle ein Shunt 14 zwischen Pfortader 12 und Lebervene 13 geschaffen. In diesen wird dann mittels eines Katheters der Shuntstent 7 eingebracht, indem nach Einführen des den Shunt enthaltenden Katheters der Shunt durch einen Schieber oder dergleichen am Anlageort gehalten und der Katheter zurückgezogen wird, wodurch sich der Shunt-Stent 7 in seine in der Figur 2 dargestellte Anlagestellung aufweitet, die, wenn er aus einer wärmebehandelten Nickel-Titan-Legierung (Nitinol, ein eingetragenes Warenzeichen) besteht, seine Hochtemperaturstellung ist. Nachdem so die akute Verblutungsgefahr eines entsprechenden Patienten vermieden wurde, kann anschließend in gleicher Weise mittels eines Katheters und eines Schiebers der Reduzier-Stent in den vorab gelegten Shunt-Stent eingeschoben werden, um den effektiven Durchflußquerschnitt bei hepatischer Encephalopathie zu reduzieren.

## Patentansprüche

1. Stent mit einem hülsenartigen Teil (2), das mit Durchbrechungen versehene Wandungen, erweiterte Enden sowie einen durch eine Einschnürung in seinem Durchmesser reduzierten Zwischenbereich aufweist, dadurch gekennzeichnet, daß der Stent zum Reduzieren des Durchmessers eines Kanals im Körper eines Lebewesens, wie insbesondere eines transjugulären intrahepatischen portosystemischen Shunts auf der Außenseite des hülsenartigen Teils (2) zwischen den erweiterten Enden thrombogene Fäden (8) aufweist.

2. Stent nach Anspruch 1, dadurch gekennzeichnet, daß das hülsenartige Teil (2) bei erhöhter Temperatur selbstexpandierend ist.

3. Stent und Anspruch 2, dadurch gekennzeichnet, daß das hülsenartige Teil (2) aus einer Nickel-Titan-Legierung besteht.

4. Stent nach einem der vorangehenden Ansprüche, dadurch gekennzeichnet, daß das hülsenartige Teil gitterartig ausgebildet ist.

5. Stent nach einem der vorangehenden Ansprüche, dadurch gekennzeichnet, daß die thrombogenen Fäden im wesentlichen achsparallel angeordnet sind.

6. Reduzierstent nach einem der vorangehenden Ansprüche, dadurch gekennzeichnet, daß der maximale Enddurchmesser geringfügig größer ist als der Durchmesser eines Shunt-Stents, in den der Reduzierstent zur Reduzierung des freien Durchflußquerschnitts im Shuntstent eingeführt wird.

7. Vorrichtung zur Anlage eines Kanals in einem lebenden Körper, wie insbesondere eines transjugulären intrahepatischen portosystemischen Shunts mit einem den Kanal offenhaltenden Shunt-Stent (7), gekennnzeichnet durch einen den freien Durchflußquerschnitt reduzierenden Reduzier-Stent (1) nach einem der vorangehenden Ansprüche.

## Claims

1. Stent having a sleeve-like part (2), which has walls provided with openings, widened ends and an intermediate area whose diameter is reduced by a constriction, characterized in that the stent for reducing the diameter of a canal in the body of a living being, such as in particular a transjugular, intrahepatic, portosystemic shunt, has on the outside of the sleeve-like part (2) between the widened ends thrombogenic threads (8).

2. Stent according to claim 1, characterized in that, at elevated temperature, the sleeve-like part (2) is self-expanding.

3. Stent according to claim 2, characterized in that the sleeve-like part (2) is made from a nickel-titanium alloy.

4. Stent according to one of the preceding claims, characterized in that the sleeve-like part has a lattice-like structure.

5. Stent according to one of the preceding claims, characterized in that the thrombogenic threads are arranged in a substantially axially parallel manner.

6. Reducing stent according to one of the preceding claims, characterized in that the maximum end diameter is slightly larger than the diameter of a shunt stent, into which the reducing stent is introduced for reducing the free flow cross-section in the shunt stent.

7. Device for applying a canal in a living body, such as in particular a transjugular, intrahepatic, portosystemic shunt with a shunt stent (7) keeping the canal open, characterized by a reducing stent (1) reducing the free flow cross-section in accordance with one of the preceding claims.

## Revendications

1. Stent présentant une partie en forme de manchon (2), des parois ajourées, des extrémités élargies ainsi qu'une zone intermédiaire de diamètre réduit par resserrement, caractérisé en ce que le stent destiné à réduire le diamètre d'un canal dans le corps d'un être vivant, en particulier celui d'une dérivation transjugulaire intrahépatique portosystémique, présente des fils (8) thrombogènes sur la face extérieure de la partie en forme de manchon (2) entre les extrémités élargies.

2. Stent selon la revendication 1, caractérisé en ce que la partie en forme de manchon (2) est autoexpansant sous l'effet de l'élévation de la température.

3. Stent selon la revendication 2, caractérisé en ce que la partie en forme de manchon (2) est réalisée dans un alliage de nickel et de titane.

4. Stent selon l'une quelconque des revendications précédentes, caractérisé en ce que la partie en forme de manchon est conformée en treillis.

5. Stent selon l'une quelconque des revendications précédentes, caractérisé en ce que les fils thrombogènes sont disposés sensiblement parallèles à l'axe.

6. Stent de réduction selon l'une quelconque des revendications précédentes, caractérisé en ce que le diamètre maximal d'extrémité est légèrement supérieur à celui d'un stent de dérivation, dans lequel le stent de réduction est introduit pour réduire la section transversale libre du stent de dérivation.

7. Dispositif pour créer un canal dans un corps vivant, en particulier une dérivation transjugulaire intrahépatique portosystémique comportant un stent à dérivation (7) pour maintenir le canal ouvert, caractérisé par un stent de réduction (1) réduisant la section transversale libre de passage selon l'une quelconque des revendications précédentes.
